# EUROPEAN PATENT SPECIFICATION

(11) **EP 3 494 503 B1**
(45) Date of publication and mention of the grant of the patent: **01.07.2020**
(21) Application number: 17781200.5
(22) Date of filing: 26.07.2017
(51) Int. Cl.: A61H 1/02

(54) **AUTOMATED ELECTRO-MEDICAL SYSTEM AND RELATED MANAGEMENT METHOD**
AUTOMATISIERTES ELEKTROMEDIZINISCHES SYSTEM UND ZUGEHÖRIGES VERWALTUNGSVERFAHREN
SYSTÈME ÉLECTROMÉDICAL AUTOMATISÉ ET PROCÉDÉ DE GESTION ASSOCIÉ

(30) Priority: 03.08.2016 IT 201600081694
(43) Date of publication of application: 12.06.2019
(73) Proprietor: Lauro Group S.r.l., 82100 Benevento (IT)
(72) Inventor: LAURO, Cosimo Luigi, 82100 Benevento (IT); LAURO, Alessandro, 82100 Benevento (IT); LAURO, Fabiana, 82100 Benevento (IT)
(74) Representative: Garavelli, Paolo
(86) International application number: PCT/IT2017/000154
(87) International publication number: WO 2018/025283

(56) References cited:
- WO-A1-2009/000444
- DE-U1-202010 007 728
- US-A1- 2005 222 523
- US-A1- 2006 277 074
- US-A1- 2008 312 041
- US-A1- 2012 209 157

## Description

The present invention is referred to an automated electro-medical system, mainly a postural system without a specialist operator's assistance or support, and to its related management method.
The method (technology) is backed by such equipment for the movement as physiotherapeutic bed type devices, that can be used for rehabilitation and curative treatments.

To date, the physiotherapeutic table is one of the most commonly used support to prevent, care and rehabilitate patients who are suffering from congenital or acquired muscular, skeletal and neurological disorders or malformations.

At present, it is possible to differentiate the physiotherapeutic tables according to use patterns: tables for medical examinations, for transferring patients, for physical manipulation and specific treatments and massages.

This distinction reveals a high supply of innovative application of technologies. It relates to several solutions for improving physiotherapeutic table's services for the patient's comfort and the user's less effort, improving its use as a whole.

In this context, the most innovative devices include such variations that may be applied to physiotherapeutic tables and that are appropriate to enable the users not to make too many physical efforts; consequently side rests for arms, wheels with increased diameter, a motorized central junction to improve patient's comfort when he is supine (in prone position) may be included among the most innovative and new devices.

Specifically, it should be mentioned a physiotherapeutic table equipped with side pressing motors adaptable to varus and valgus deficiency's treatment which is already in existence, as described in patent NA2013U000017.

Similarly, it should be mentioned a preexisting bed for massage therapy equipped with a goniometer uncoupling/coupling device, to treat patients who are suffering from spinal disease, as described in patent TS2010A000004 (IT1401228).

For patients treated with direct manipulation, it should be mentioned a preexisting semi-automated postural bed type [1], equipped with manual levers which can be operated by patients too, so to facilitate the therapist during his treatment and to ensure the continuity of the therapy.

Notably the postural bed is very effective to perform an accurate muscular stretching, to recover the physiological range of motion, to achieve relaxation postures helpful in spinal pains, to get an optimal primary position, and then to practice for stabilizing the rachis, but only under therapist expertise. Besides, this pre-existing postural table is not allowing for a reduction in therapist's assistance and can treat only a limited number of diseases.

Musculoskeletal system disorders are divided into para morphisms, i.e. locomotor system's reversible attitudes and dysmorphisms, which are locomotor systems' structured diseases; in the former case, they are reversible and treatable postural habits with a high probability to develop into severe and irreversible conditions. If that is the case, continued treatment or even surgical interventions would be needed.

In the latter case, they are real structured diseases which are intractable according to official medical literature; in the most severe cases, a particular surgical intervention would be needed.

The importance of such diseases has been well known for decades. In practice, however, scientific investigation has been focusing on disease diagnostic phase only, and a lot of devices has been flourishing, mostly able to give merely an additional acknowledgement to the clinical diagnosis.

The therapeutic step is based on a methodology that inevitably requires a very qualified staff, such as a specialized operator who is in charge of the entire therapy, by his expertise and his title: at times, he can only rely on outdated mechanical equipment.

Presently there is an absence of a completely automated therapeutic system, which is entirely customizable and aimed at the treatment, the analysis and the observation of the issues about the locomotor system and the vertebral column.

The present invention aims at solving the problems as mentioned above on the previous technique since it provides an automated electro-medical system unable to submit simultaneously one or more patients to a particular dedicated therapeutic path.

A further aim of the present invention will be to provide a method of management and automation able to customize the abovementioned electro-medical system to some patients who are suffering from particular different diseases.

More invention's aims and advantages other than the ones above will be described hereafter and will be achieved with an automated electro-medical system and its related method of management as outlined in each of the corresponding separate claims.

The scope of each independent claim is to provide preferred achievement patterns and non-trivial variations of the present invention.

It is intended that all the attached claims will be an integral part of this description.

It will be apparent that any number of variations and amendments (i.e. those referring to format, scale, requirements and elements with an equivalent functionality) may be provided, without departing from the invention's field of protection, as it can be seen from the enclosed claims.

The present invention will be well described by some preferred achievement patterns that will be provided by way of example, but that will be not limited to them, concerning the enclosed pictures:
- The pictures 1-5 show the corresponding perspective views of the devices that form an achievement of the automated electro-medical system according to the present invention.

By reference to the pictures, an automated medical system which is based on the present invention can treat, cure, analyze and monitor a plurality of medical diseases related to the spine and the musculoskeletal system such as scoliosis, scoliotic attitude, lordosis, hyperkyphosis, arthritis, herniated disk, muscular atrophy's degeneration diseases, geriatric articular and muscular pathology, prevention of muscular and tendinous acute and chronic diseases, knee valgus, knee varus, etc., even in absence of a specialized operator.

The system based on the present invention includes a minimum of five devices acting in synergy to support medical treatment, to which one or more patients are being subjected at the same time, enabling them to perform a therapeutic path by using all the devices. The devices act in synergy to treat specific musculoskeletal system disorders, some of which should have been treated using surgery, through any specific therapeutic protocols, even without a qualified staff of operators:
- a first device 1 provided with an automated and interactive postural table;
- a second device 2 provided with an automated postural and interactive bench;
- a third device 3 for an automated and interactive alignment of the spine;
- a fourth device 4 for automatically and interactively hydrating the intervertebral disks; and
- a fifth device 5 for an automated and interactive alignment of lower limbs.

Using all the five methods mentioned above, the patient will perform a therapeutic path to treat all the particular musculoskeletal system problems, some of which might need a surgical procedure.

All the devices of the methodology are necessary, since every single one of them enables the implementation of some therapies, by performing specific therapeutic protocols. In that way, they make every muscular chain work in many completely different ways (open kinetic chain, closed kinetic chain, mixed kinetic chain).In doing so, using all the devices in the same therapeutic session becomes essential.

Thus, the standardized therapeutic path succeeds in becoming comfortable for the operator to apply (his tasks being reduced to those of monitoring and control) and for the patient also.

Especially the first device 1, the automated and interactive postural table, is the necessary implement of the method; it may be considered the "core" of the therapeutic session.

It is shaped like a bed, hence a device the patient is "familiar" with; this solution could increase his compliance, especially if the patient is an older person with severe diseases or if he is a child.

This device's unique features and innovation are considerable:
- it is equipped with an automatic movement (handling) since it executes the inputs of a dedicated software (explained below).Since the first device 1 works in an entirely autonomous way, the patient may relax, bypassing the invasive involvement of an operator;
- the operation of the two main motors specially designed on purpose, which has been calibrated to a particular speed parameter (mainly examined and clinically tested), provides the extension of the posterior muscular chain and the relating muscular bands, in a slow and gradual (progressive) way. This action provides to avoid the inhibitory control partly by neuromuscular molten, which would prevent the maximum extension;
- the software is composed of several phases, each of which let the patient perform any specific therapeutic protocols, leading him into the therapy. The software provides the patient information on specific positions he will take to let the device operate on his diseases to achieve the maximum possible levels at that given moment and in a specific way for each protocol. There are different protocols, and all of them enable the device to operate over the whole body, from the head to the lower limbs, from the perspective of an overall therapy (this particular feature exists in all the present invention's devices). The method of the present invention may be considered one of the "overall" existing methods, since it involves the whole body, providing it with a correct posture while it is working, and without any offset (this is another particular feature of the present therapeutic method). This particular feature represents the different main point of the present methodology from other existing methods, especially from manual physiotherapeutic techniques which provide the body to find out any offset. In doing so, they could abort or minimize therapy's effects and final effectiveness;
- this first device 1 is equipped with two more side motors, which work with (in) a specific (particular)positioning. These two positioning motors serve to compress legs from the side to realign them, using as an addition a cylinder-type spacer placed between the knees (in case of *valgus knee* disease), or between the ankles (in the event of *varus knee* disease), while the other two main motors work on extending muscular chains, both trunk and lower limbs ones. The combined action of the four motors ensures that the final result will be a specific action on chains and fascias, both lower limbs extending chains and cruciate fascias. Apparently, lateral compression is fixed by the operator and performed to the knees, in the presence of varus knee deficiency and, vice versa, to the malleolus, in the presence of valgus knee gap;
- the software of the first device 1 is composed of six therapeutic programs (specified below). Two of the programs are unique for varus and valgus knee's treatment, two further ones are appropriate programs for first therapeutic paths, both for particular therapies to patients with severe musculoskeletal system or neurological diseases, cardiopathic and/or patients being suffering from the effects of any surgery, and it can be suitable for patients with severe muscle and fascias retractions. One of the programs provides a specific action on hip's rotator muscle (particularly musculus piriformis), thus working out on sciatic nerve's diseases (compression from musculus piriformis). One of the most frequently used programs is the one for the treatment of musculoskeletal system deficiencies. Eventually, an additional program is being used for overall muscular strengthening and for specific lower limbs' one, when in correct posture; and
- the first device 1 makes the therapies and customize every parameter for each patient. This "customization" is undertaken through a preliminary test to detect his maximum level parameters: the operator records every patient's specific threshold, to enable the therapeutic software to process data and make an individual therapy for each of them. Technically, the procedure consists of letting the device perform its necessary manual movement (namely, seat and backrest lift up alternatively), with operator's assistance, while it's the patient who shall indicate him to "stop" when in his threshold. At this stage, the device records any particular parameter for every single protocol. The software incorporates these maximum levels and processes associated algorithms, so developing specific parameters for the treatment. As therapy proceeds, the operator will repeat the test, to enable the software to calibrate the new parameters and set new algorithms for the treatment, detecting any improvement and making the first device 1 work in progression (generally it should be done every ten sessions of therapy). The test for the detection of patient's maximum levels is the only time the assistance of an operator is required.

Notably, referring to Picture 1, the first device 1 is made up of a combined mechanical, electronic and computerized system; it is similar to a medical table and comprises:
- a base (101);
- a backrest (102) connected to the base so that it can rotate (101);
- an abutment for lower limbs (103) attached to the base (101) and the backrest (102) so that it can rotate;
- the base (101) is specifically a metal box with an actuator to move the backrest (102), an actuator to move the abutment for lower limbs (103), an electronic part, (namely a card and some components) contained inside;
- a carriage for feet (104) operatively connected to the abutment for lower limbs (103);
- side pressing motors (105) to hold the patient at his ankles or knees, which are attached to the abutment for lower limbs (103) along a sliding carriage (106); and
- two side rests for arms (107) connected to the backrest (102).

The first device 1 also comprises following accessories :
- a central pillow (108) placed next to the connection fulcrum among base (101), backrest (102) and abutment for lower limbs (103);
- two upper extendable handles (109) connected to the backrest (102) on the opposite side from the central pillow (108);
- six adjustable resistance bands (112), connected to each other as follows: two resistances are connected between central pillow (108) and abutment for lower limbs (103); two more resistances are connected to the upper side of the backrest (102); the last two resistances are connected to the carriage for feet (104); and
- a personal computer, (preferably an All-in-One type, equipped with managing software and webcam), fastened to a bracket connected to the abutment for lower limbs (103); the personal computer is being used for controlling via software both the table movements and the particular therapeutic path for each patient.

At present, the only way to stretch muscular chains and their fascias are performing postural floor exercises or using manual benches, which require a therapist expertise to check the correct execution of the exercise, otherwise it might not be effective.

The first device 1, on the other hand, lets the user operate independently and does not require any specific operator.

This is due to a software equipped with six different programs to treat musculoskeletal system diseases. The therapies may be customized, since a test to determine each patient's threshold is made during his first session; it consists in a detection of all his specific parameters to be recorded in the initial situation and to be adjusted over the course of therapy, to improve the work of the device.

Every single patient's specific detection of his parameters will let the software to process special algorithms, calibrating motors' movement in such a way as to allow the therapy to follow every single patient's setting. The software of the therapy, according to the particular selected program, acts through several steps or phases. During each step, the patient is indicated the position and his particular therapeutic protocol to perform, through a picture and a textual description.

It has been calculated both movement time and rest time for each phase so that the performance of the stretching exercise and the concerning pause can be optimized in line with human physiology. Eventually, for the protocols of trunk and upper limbs, namely for the movements of head, neck and arms, the software, along with both picture and textual description, by default activates a webcam which has been positioned at a certain distance on the PC's bracket, so the patient can verify if he is correctly performing the protocol, since the webcam projects his image on a monitor in front of him (visual feedback).

As a general outline of the first device 1 we may recap as follows:
1. it is a tool that allows performing maximum stretching on posterior muscle chain both in open and/or mixed kinetic chain, through an adjustment of the guide sliding on the footrest carriage (for foot). This is a dynamic action due to the low-speed motors which raise the ground planes up in a combined mode. The performance is not randomized but predefined, according to time of execution and rest by the software;
2. it is a device which can be set off by the patient himself through an R-FID card and a player. Each card shall associate and identify every patient and store patient's tests (i.e. his specific parameters), his data, his medical records, the number of his healing sessions, his report, etc.). The patient can also select the appropriate program for his disease with a check mark on PC's touch screen; all can be done without any assistance by an operator;
3. it allows the treatment of more patients at the same time since there are many tools available;
4. patient's compliance;
5. quick and more efficient achievements than in bodyweight or stationary execution;
6. the therapy provides a slow and gradual movement (dynamic). It enables the realization of significant results since it succeeds to inhibit partially extension osteo-tendon receptors (the Golgi tendon receptors).

The first device 1 will treat musculoskeletal system and spine diseases:
a. head (retraction of laterocervical muscles);
b. lower limbs (treatment of dysmorphisms: varus knee and valgus knee); and
c. spine (scoliosis, hyperkyphosis, hyperlordosis, etc.).

The management software of the first device 1 may be considered an integrated application software inside the device itself, fit for facilitating the therapeutic activity. Specifically, it deals with the management of the device by the patient, since it arranges for the patient to perform all the activities and therapeutic protocols correctly, thus reducing physician or operator's involvement in a mere supervisor.

The software of the first device 1 is an automated system fit for treating all diseases for which the system has been implemented. There are several programs provided to treat all the correctly identified conditions.

Several medical statistics agree that the musculoskeletal system and spine diseases are the most common pathologies being suffered by the human race.

To intervene with non-invasive therapies (as opposed to surgical intervention), these pathologies need to be considered very carefully, since they could be treated with this method. Moreover, the methodology could also lead to prevent that these diseases may degenerate in more severe disorders.

In the last years, technology succeeded in improving diagnosis, whereas therapy has been developing in different methods supported solely by qualified operators; consequently very few patients may be treated and the therapeutic results are limited.

The first device 1's managing software main aim is to remedy this limit. Indeed it would be:
o highly diffusible;
o easily comprehensible by the patients; and
o usable with a minimum staff required.

Operators' lack in expertise could be compensated due to high technological standards of the device, and the operator could start treating patients immediately, to achieve results from the outset while completing his expertise.

About the second device 2, unlike the old segmental and passive stretching methods with limited clinical results, the global active, slow and progressive muscular lengthening has been proved to be more efficient, since it works on different muscular chains simultaneously.

The most used method, known as "global active stretching" comes from Souchard Global Postural Reeducation (RPG) and has been applying to treat static muscular retractions. Static anterior muscle chain is composed of scalen and intercostal muscles and provide chest stability, diaphragm, up to psoas, muscles of the anterior legs and abductor's muscles.

The retraction of this chain has the effect to pull the head forward, bend the backstroke and round the shoulders; the abductor's muscles shorten, pulling knees together, legs anterior muscles rotate inwards, the feet show up flat (fallen arches).

The Global Active Stretching is based on the execution of any selected positions, called "postures", that have to be maintained few minutes to stretch retracted muscular chains, which have been detected in advance through a morpho-postural test on the patient.

The second device 2 enables to maintain therapeutic postures of Global Active Stretching more efficiently and comfortably to achieve targets in a motor, physical, sport and prevented activities, vibrant health, wellness and fitness for a better quality of life.

The second device 2 is a motorized tool. The patient himself may monitor the movement through a remote actuation. The monitoring by the patient proves to be very helpful for several reasons:
- the patient compliance;
- a total control of the extension of the muscular chain. The total control allows the patient to achieve the highest in that session; and
- the gradual and progressive extension, both in that very session and in the following ones, allows him to deliver results faster, without any offset from other parts of the body and, most importantly, without side effects.

Regular use of the second device 2 could lead to several beneficial effects, as follows:
- stretch the retracted anterior muscular chains;
- improve range of motion in extension of head and trunk;
- align pelvis;
- reduce or remove pains originating from an incorrect posture; and
- support postural rebalancing.

The therapy is intended for:
- thoracic hyperkyphosis, lumbar and cervical rectilinearization treatment;
- hernia and/or low back pain prevention; and
- spine small joints (facet joint), arthrosis (arthritis) prevention and treatment.

At the present time, stretching muscular chains and fascias is possible only executing postural exercises which require a therapist expertise to check the correct execution of the exercise; otherwise, it might not be effective.

The second device 2, on the other hand, lets the user operate independently and does not require any particular operator.

That is due to a remote control that makes the two actuators move. The first actuator allows the distal part of the anterior chain, namely tiptoe, to extend at most. The second actuator of the movement of the backrest is always controlled by the patient. This allows him to reach the best performance in stretching his anterior chain.

Once the patient has reached his ceiling, he will maintain the position for 30 seconds, and then he will get back to the starting position and rest.

The more the patient goes on repeating the exercise, the more he succeeds in extension.

The second device 2 allows the extension of the anterior chain in an entirely progressive way and, more importantly, without any pain.

Being the patient confident he may start from an entirely relaxed position up to reach his threshold, shall ensure him that achievements can be fast while respecting his compliance.

Referring to Picture 2, the second device 2 is shaped like a bench and comprises:
- a supporting frame (201) equipped with a sliding guide (202) for supporting pillows (203) made from rubber;
- a base, which is a metal box composed of a first half-carter (208) and a second half-carter (209), and which contains at least one actuator (205) to move a backrest (211), at least one actuator (204) to move the rubber supporting pillows (203) inside; a control unit, to which a remote control is fastened, is inside the carter (208,209). It is expected to control the two actuators (204, 205), the backrest (211) being made to operate through a motor inserted in the base (208, 209);
- a seat, (210) which mirrors the backrest (211) versus the juncture, is stationary;
- the actuator (204), fastened to the supporting frame (201), moves the rubber supporting pillows (203), making them slide alternatively;
- a headrest (213) connected to the backrest;
- a roll carrier (206) attached to the headrest (213) ;
- a handle (207) connected to the backrest (211)
- two adjustable coasters (nubs) (214) equipped with plugs (212); and
- a mobile ankle-carrier (215) provided with an adjusting knob (216).

As a general outline of the second device 2 we may recap as follows:
1. it is a tool to stretch the anterior muscular chain in a correct posture with no offsets;
2. it is a tool that can be set off by the patient, without any assistance of an operator;
3. it allows the treatment of more patients at the same time since there are many tools available;
4. quick and more efficient achievements than in bodyweight stretching exercises;
5. patient's compliance.

Several head and trunk disorders, (neck pain, back pain, low back pain, lumbar radical pain) and lower limbs diseases (varus knee and valgus knee) are the results of posterior mio fascial kinetic muscular chain's retraction.

Consequently, cervical, deep and superficial spinal, pelvis-trochanter, ischial crural muscles, gluteus, triceps surae and popliteus muscles can benefit from an advanced, slow and progressive methodology based on posterior active global stretching.

Malformations, pathologies and notably para-morphisms of musculoskeletal system are most frequently caused by a wrong alignment of bone segments. To prevent and treat those disorders is crucial to align the bone segments.

The several methods of Functional Rehabilitation pursue that aim.

Referring to Picture 3, the third device 3 comprises:
- a motorized chute (301), possibly equipped with a sliding-preventing rubber plane (309) and a supporting frame (302);
- a containing structure composed of a carter (312), a curved carter (303), with related small adjustable feet (315);
- an actuator inside the containing structure;
- a monitor (311);
- a quadri-visor (310);
- four cameras (307) which allow the patient to obtain the alignment of the musculoskeletal system autonomously;
- monitor holder (311);
- fixing lever (304);
- video cameras (307);
- one side (lateral) curved camera holder bracket (308) ;
- one quad vision (310);
- one front panel (312);
- a monitor (311);
- a quadri-visor (310);
- four cameras (307) which allow the patient to obtain the alignment of the musculoskeletal system autonomously;
- monitor holder (304);
- fixing lever (305);
- video cameras (307);
- one side (lateral) curved camera holder bracket (308) ;
- one quad vision (310);
- one front panel (312);
- a switcher (313);
- a safety lock (314);
- a lateral panel (315);
- one lateral curved video camera holder bracket (316) ;
- videocamera (307) height control (317);
- two buttons for actuator's movement control (306) ;
- one handle (318); and
- a blockade ball (319);

The alignment shall be carried out progressively, according to every single patient's benefits:
- re-aligning the lower limbs;
- stretching the retracted posterior muscular chain;
- repositioning a correct asset of pelvis, spine and lower limbs simultaneously; and
- reduce or/and remove pains of spine originating from an incorrect posture.

The method is indicated to treat:
1. paramorphism of the spine;
2. hyperkyphosis, hyperlordosis, scoliosis;
3. diseases of the disc (protrusion, hernia); and
4. varus knee and valgus knee.

At present, an alignment of several body areas to solve a large number of pathologies of the musculoskeletal system has been performing using therapeutic techniques based on exercises according to different methods, as Mezieres', Souchard's, etc.

These methods require a therapist expertise to check the correct execution of the exercise; otherwise, it might not be effective.

The third device 3, on the other hand, lets the user operate independently and does not require any specific operator.

This is possible since the third device 3 is equipped with a motorised inclined plane (301) (through an actuator) that allows the extension of the tibial tarsal area and is led by the patient himself through a remote control. Also, there are four cameras: the first one is located at the top of a curved pole, the second one is located in the middle of the pole, the third one is attached to the lateral prolongation, the last one is located under the monitor. The four cameras allow seeing in real time the position of the patient's body from different perspectives and angles, particularly, the first camera shooting the spine from above, the second one shooting the lateral vision of the torso, the third one shooting the lateral view of the whole body, and the fourth one shooting the precise alignment of the lower limbs.

The inclined plane (301) is managed by the patient and allows him to start from a relaxed position. To the extent that the alignment improves, the patient is going to lift the inclined plane and increase the flexion of the tibial tarsal area, thus making the protocol and therapy harder, with the result that he will progressively improve

When the patient reaches his threshold, he will maintain the position for 30/60 seconds; then he gets back to the initial position and rest.
The more the patient goes on repeating the exercise, the more he succeeds in extension, while maintaining all the areas of the body aligned.

The third device 3 results in the gradual and progressive extension of the posterior chain in a correct alignment and, mainly, with no pain.

Being the patient confident he may start from an entirely relaxed position up to reach his threshold shall ensure him that achievements can be fast while respecting his compliance.

The outlines of the third device 3 are the following:
1. it is a tool to stretch the posterior muscular chain in a correct posture with no offsets of any body areas;
2. it is a tool that can be set off by the patient, without any assistance of an operator;
3. it allows the treatment of more patients at the same time since there are many tools available;
4. quick and more efficient achievements than in bodyweight stretching exercises; and
5. patient's compliance.

The fourth device 4 supports the hydration of the intervertebral disks, so to ease the load and to make the spine more flexible.

The fourth device 4 is a motorized tool with remote control, so it allows the patient to regulate the strength of the therapy autonomously till his threshold.

Gradually the patient will reach increasingly high ceilings time, period, power.

The therapy is intended for:
- all the pathologies related to dehydration of the intervertebral disks, namely protrusion and hernia;
- the treatment of thoracic hyperkyphosis, hyperlordosis;
- the prevention of hernia and/or low back pain
- the treatment and prevention of arthritis of the spine small joints; and
- stretch the spine when in extension.

From the age of 25, the intervertebral disks begin to deteriorate. This is because it is not blood that immediately fuel them, the interstitial fluid does: this process occurs during the night in lying position. The intervertebral disks absorb interstitial fluid like a sponge upon lying down during the night, while, during the day, they leak out when standing and walking. This is the reason why the disks end up suffering a dehydration and, over the years, become less flexible, especially in those doing arduous work, for an excessive loading on their spine.

At the same time, in the outer layers, the connective tissue, also known as collagen, that covers the nucleus polposus of the disk like a net, loses its sturdy frame. For this reason, whether under strain, there may occur some little tears; consequently the soft inner part of the disk prolapses (herniates). If the disk doesn't get sufficient nutrient fluid, it begins a degenerative process, and the disk will get smaller and lose its flexibility. The spine small joints will be crushed, and back pain will occur: it might be local, if under strain, and lateral, coming up from the hips.

The pathology gets worse in presence of spine disorders, the most common of which begins at a young age (hyperkyfosis, hyperlordosis).

Referring to Picture 4, the fourth device 4 comprises two pillars (401,408) connected to a plane with a curved pillow (410); inside the carter (408) of the right pillar an actuator (404) is inserted. The actuator that is actuated by a remote control (406), gradually allows lifting the plane (410) connecting the two pillars (401,408), till its maximum height (70 cm). The patient will place his body area between shoulders and lumbar vertebrae on the plane (410) connecting the two pillars (401,408) when it is at its minimum height. Starting from this position, he will slowly begin to lift, using the remote control (406), till the maximum height he can bear, maintaining the position until he feels comfortable. Then, the patient will gradually lower the plane, using the remote control (406), till the starting position. He will repeat the exercise many times, till a progressive flexibility of the spine is obtained, with benefits for inter-vertebral disks.

The device also comprises:
- one carter |(|[2] folle) (401);
- one structure (403) of the plane (410);
- the two pillars (401, 408) are connected and stabilized by an iron frame and by a tubular curved bracket (402) as a conjunction of the upper part of the pillars (401, 408);
- a control unit (405) with a bracket; and
- an adjustable foot (407).

At present, the spinal mobilization in extension is provided by a therapist, who treats it manually or using a curve ladder or a big medicine ball. This equipment always needs therapist's support, at least for supervision and safety.

The fourth device 4, on the other hand, lets the user operate independently and does not require any specific operator.

This result may be obtained because the device 4 is equipped with a remote control (406) that makes the actuator (404) move. The actuator is inserted on one of the sides of the fourth device 4. In this way, the patient can adjust the maximum level of height as long as he feels no pain, succeeding in recover gradually and progressively flexibility without any pain.

Being the patient confident he may start from an entirely relaxed position up to reach his threshold shall ensure him that achievements can be fast and efficient while respecting his compliance.

The outlines of the fourth device 4 are the following:
1. it is intended to provide flexibility to the spine in extension, and it can be controlled by the patient;
2. no operator support is required;
3. it allows the treatment of more patients at the same time since there are many tools available;
4. quick and more efficient achievements than in bodyweight stretching exercises are possible; and
5. patient compliance is guaranteed.

The fifth device 5 can be considered a useful and additional tool to the 1 to 4 devices of the method. Specifically, it adds extra benefits the other 4 devices cannot supply.

Referring the picture 5, the fifth device 5 comprises:
- a supporting structure (503) with possible adjustable small feet (508);
- a double chute (502), on which the patient is placed standing when the base (501) is horizontal, two separate planes (502), one for each foot, being controlled by a push-button panel which activates an actuator placed below the two planes (502). The actuator lifts the two planes (502) up and/or down, forming an acute angle by rising upwards or descending downwards. Thereby the patient's ankle is strongly stressed, when in prone or supine position, according to whether the plane (502) is lifting upwards or downwards.

The fifth device 5 is a motorized tool equipped with remote control, so it allows the patient to regulate the strength of the therapy autonomously till his threshold at that moment.

Gradually the patient will succeed in increasing higher ceilings of time, period, power.

The therapy is intended for:
- axial lower-limb alignment pathologies (varus knee and valgus knee (malalignment of limb segments);
- pelvic imbalance;
- upward scoliosis type attitude;
- hindfoot varus; and
- hindfoot valgus.

At present, lower limbs' axiality deficiency and hindfoot pathologies are treated by the prescription of splints to put during the night, or arch supports to slip in the shoes. Both solutions are extremely disappointing as for the results as for the weak patient's compliance since most of them are very young (from 7 to 15).

The fifth device 5, together with the devices from 1 to 4, not only finalizes the therapy of the axiality and the torsion of the lower limbs but adds a particular treatment to a gradual hindfoot (calcaneus) realignment, according to the little patients'limits. At the same time, the patient will also perform specific protocols to align pelvis and trunk.

The outlines of the fifth device 5 are the following:
- it is intended for the re-alignment of lower limbs (varus and valgus knee), through a therapy led by the patient, who can adjust the angle of the planes making therapy harder;
- enables the treatment of varus and valgus hindfoot;
- it provides the ankles to get flexibility when in prone/supine position;
- it does not require any qualified operators;
- it allows the treatment of more patients at the same time since there are many tools available;
- quick and more efficient achievements are possible, compared to all of the other non-innovative tools (splints or arch foot); and
- patient's compliance is guaranteed.

As recognized, muscle chains are constituted by a series of muscles all adjacent one another; each muscle links in a chain that involves the whole body. Muscles of the same chain work as one family. The main muscle chains are the main posterior chain; main anterior chain; inspiratory chain; upper shoulder muscle chain; anterior and inner shoulder chain; anterior arm chain; hip anterior and inner chain; lateral hip chain.

Each of the 5 devices perform particular therapeutic protocols. They exercise muscle chains in open kinetic chain, where the muscular chain's distal end is free in the movement with no boundaries, i.e. a leg during ambulation in the oscillating phase, or an extended leg when seat, or an arm movement when throwing something, etc.; in closed kinetic chain, where the distal end of a muscle chain is fixed and tied, i.e. during ambulation when the foot is landing on the ground or like someone's legs who is lifting a weight, etc.; or in mixed kinetic chain, where the external distal opposition may be less than 15% of its maximum opposition (slightly restrained open chain), or more than 15% of its maximum opposition (strongly restrained closed chain), consequently limiting the range of movement of the patient's musculoskeletal system.

Consequently, the efficiency of the whole system of the present invention, starting from the device 1 to the device 5, is clear: the entire therapeutic path may be standardized, so making the assistance of an operator easier, since his tasks will be reduced to supervision and control. In this way, the protocol is guaranteed to be safe, standardized and independent from an operator's professional skills and even from his presence.

The system described above has its privileges:
- fully autonomous;
- therapy automated program;
- management software;
- patient's data processing and therapeutic outcomes;
- staff zero cost;
- minimum training required for an operator;
- immediate treatment on patients;
- results in a very short period;
- treatment of more than one patient at the same time;
- new robotic scheme equipped with a user-friendly software interface and an embedded system, reconfigurable to data monitoring and acquisition;
- direct interaction between patient and the system;
- ongoing monitoring;
- real-time control of the outcomes;
- interactively current information about the therapeutic protocols; and
- data processing and storage through the management software.

Future integrations for the devices from 1 to 5 will be possible, as follow:
- for the first device 1, current management software is being projected to develop into a more complex one. In the new one, the current therapeutic standardized programs will be replaced by a dedicated programming. Physician will be able to select among lots of protocols, all grouped into categories and, according to his selection, software will develop a proper therapy by default;
- still, for the first device 1, a dedicated variation is being projected on a particular table for the scoliosis' treatment (optional), by adding some linear pressoreceptors on the backrest of the table. During the therapy, pressoreceptors act to balance chest cavity's rotation, through a dedicated software;
- still, for the first device 1, a specialized variation is being projected on a specific table for the spine's treatment (optional), by adding a long curved facility to support the spine. It will be attached to backrest's surface, and it will be adjustable in height, as necessary.
- for the second device 2, pressoreceptors will be added along the backrest,up to the lumbar area of the patient's spine. A further addition of a dedicated software will allow detecting the gap between the lumbosacral region and the backrest, accordingly reducing its descent, thus preventing the patient from arching his back. The device will be provided with a little monitor at the top, next to the head, so enabling the patient to check his back's gap from the backrest and check the angle; and
- for the third device 3, the current quad visor, whose primary purpose was to project the image of the body from different perspectives through the four cameras installed in particular areas of the device, will be replaced by a dedicated software. The program, through markers placed in specific areas of the patient's body (back, pelvis, shoulder, hip, knee and tibial-tarsal region), will detect and project body's position on the monitor, so the therapeutic protocols will be performed more accurately.

The method for the medical system above can be managed as follows:
The main operational phases are:
1) the patient gets on the step. The operator places some markers on his spine, pelvis, shoulder, knee, tibial-tarsal areas;
2) the software starts to operate: in the beginning, it shall indicate to the patient, through a picture and a textual description, his particular therapeutic protocol to follow (this phase should be of a fixed duration); then, software shall start the reproduction of patient's body alignment automatically, virtually reconstructed in real time through the markers, to make the therapy more efficient and accurate; and
3) a dedicated program on scoliosis' treatment is being added to the device's comprehensive software. Some more markers shall be provided to be placed in specific areas of body's patient. The markers will allow to reconstruct and virtually play in 3D his body projection. The software will lead patient to perform his planned therapeutic protocol.

To manage the first device 1, a sophisticated and innovative technology has been created to treat the spine and the skeletal muscle system, resulting from years of experience in the postural field of Medicine, and from clinical investigation and implemented technological research.

This method simplifies all the therapeutic techniques: this shall be granted through the equipment where the patient lies down.

The method also manages users' data and performances through any diversified and customized protocols.

Through RFID technology it is possible to check user's identity and adjust protocols to his specific parameters.

Hereafter the programs of the method:
- **Rehabilitation:** it is intended for elderly patients and for the treatment of such pathologies preventing natural movements (joint replacement, degenerative diseases, arthritis, etc.), for those who require bed rest, for Parkinson (combined with proprioceptive therapy and fkt), muscular hypertonia. Rehabilitation can also be divided into:
   1) **Rehabilitation for beginners:** it is intended for all those patients who require bed rest, elderly hypokinetic patients, neuromuscular diseases, posterior chain muscular hypertonia, hamstring muscles stiffness,ictus,contracture caused by wheelchair, paralysis,post-traumatic rehabilitation, post-surgical orthopedic rehabilitation, lower limbs' microcirculation pathology, lower limbs' limited lymphatic drainage (combined with fkt and kinesiotaping), slight arthritis of the hip joint (coxarthrosis), mild arthrosis of the knee (gonarthrosis), rehabilitation for hip replacement (six months immediately after surgery, in presence of replacement anti-dislocation provision (maximum hip's flexion 85°, no legs crossed provision), knee replacement rehabilitation, severe low back pain, severe lumbar radical suffering;
   2) Advanced Rehabilitation: Rehabilitation for hip replacement (after the sixth month, when replacement anti-dislocation provision is over), knee replacement rehabilitation, chronic low back pain, chronic lumbar radical pain.

Other programs are:
3) **FKT Therapy,** intended for the treatment of scoliosis, scoliotic attitude, hyperlordosis, hyperkifosis, shoulder diseases, spondyloarthrosis, neck pain, reactilinearization of the neck, chronic lumbar radical suffering, chronic lumbar cruralgia, discopathy, mild coxarthrosis, mild gonarthrosis;
4) **Valgus knee Therapy,** intended for the treatment of valgus knee (all grading I-IV);
5) **Varus knee Therapy,** intended for the treatment of varus knee;
6) **Power Treatment,** intended to tone trunk and upper limbs' muscles (in correct posture).

Each of the six programs are user-friendly through a touchscreen pc, and easy to use by both the operator and the patient. They prepare and develop several therapeutic protocols, for a total time of no more than 45 minutes for each session.

When the first device 1 starts to operate, it shall be calibrated. The operator initiates the procedure through the touchscreen pc, by getting the operator's card read by the RFID player.

User's login may be done in two ways:
- username and password selected during the registration; and
- RFID card: the card associated to the patient during the registration shall be got closer to RFID player. Two scenarios are possible:
   ∘ the patient will get his card closer to the player and, after an acoustic signal meaning that reading has been completed, proceed to select the therapy;
   ∘ the operator will get his operator's card closer to the player, gets an acoustic signal, and within 30 seconds get the patient's card closer to the player too. After a second acoustic signal, he may start the ceilings' test to record therapy's parameters of the patient; and
   ∘ the administrator will get his master card closer to the player, get an acoustic signal and perform the required procedure for his support.

The device can be also managed manually, but this specific access is limited to the admin/physician's Master card. It may be performed through a particular procedure that can be decided only by the administrator and specifically dedicated to every single patient. For this procedure neither user's data nor any maximum limits' test are required, since every action of the first device 1 will be set by the administrator. Through the screen he may lift, stop or drop both the backrest and the seat, and regulate the angles of the table. All the movements can be performed through corresponding buttons on the board of the screen interface, or simply using the single commands "forward" and "back".

In short, three management levels have been provided:
1) MASTER CARD (a complete access to the different tasks: MANAGEMENT, CALIBRATION, TEST, MANUAL MODE);
2) OPERATOR'S CARD (CALIBRATION AND TEST);
3) PATIENT'S CARD (READING ONLY).

The ceilings' test can be started, but it can be done only with the operator's support: in this phase he will get his Master card closer to the player first, and then he will repeat the same with the patient's one, within 30 seconds.

The ceilings' calculation comprises 8 steps, during which the patient may check his own maximum limits. At first, the therapeutic table indicates the correct body posture, then the backrest or the seat begin to lift. When the patient is at his breaking point, the operator will stop the uplift through the STOP button on the table. By stopping the backrest or the seat's uplift, the value is displayed next the corresponding step.

The corresponding data will be used during the protocols as the very limits to their patient's performance.

This transition is required for new users, when they are registering at the system the first time; it is optional for the users who are already registered, even if the operator can decide new tests for them to monitor their performances' advancements: the more the tests, the more the therapy's efficiency.

User Identification Mode is necessary in case you register a new patient in the table's database, or if you have to modify patients personal data. It can also be used by the administrator to change every single patient's residual therapies.

The access to the **Rehabilitation for beginners** mode requires only user's card to come closer the player. User can choose the program, through the pc touchscreen, and by getting his card closer to RFID player, the program will start by default.

The Rehabilitation for Beginners' mode, unlike the Advanced Rehabilitation program, provides only one body's position, the lying position. The method is composed of 5 steps, each of them including one or more movements of the backrest or the seat. During the Rehabilitation for beginners mode, the patient may ask for a video to be screened all over the session.

The access to the **Advanced Rehabilitation** mode requires only user's card to come closer the player. User can choose the program, through the pc touchscreen, and, by getting his card closer to RFID player, the program will start by default.

The Advanced Rehabilitation's mode allows a complete muscle chains' stretching through different table's movements, combined with user's position shifts. The provided positions are the following:
- LYING POSITION : user is lying on the table with his arms straight and palms of his hands facing up;
- FROG'S LEGS POSITION: from the starting position as described above, user will fit his feet together, and open up his knees;
- RIGHT LEG ON LEFT LEG POSITION and viceversa: user will cross his legs, his right ankle fit together his left knee and viceversa.

The method is divided into phases or steps, each of them including one or more lifting of the backrest or seat.

The access to the **FKT** mode requires only user's card to come closer the player. User can choose the program through the pc touchscreen, and, by getting his card closer to RFID player, the program will start by default.

In the REHABILITATION mode you will notice that all cycles of therapy are provided only through the movements with the first device 1: for the FKT Therapy mode, user will be asked to perform any movements together with the liftings with the first device 1.

When the phase 8 starts, a video is shown on the monitor through the webcam located on the table: video will be removed only at the end of the session. Phases of the Therapy FKT from 8 to 14 will be repeated in cycles 2 times.

The access to the **Power Treatment** mode requires only user's card to come closer the player. User can choose the program through the pc touchscreen, and, by getting his card closer to RFID player, the program will start by default.

As in the previous FKT Therapy, the patient is required to perform movement combined with the table: but this method differs from the previous one, since its aim is to reinforce muscles in a correct posture through protocols performed using several resistances, that are placed in different areas of the device.

The exercises and the movements for the Power Treatment mode are the same as those in FKT mode up to phase 14.

At the beginning of the phase 2, a video is shown on the monitor through the webcam located on the table: video will be removed only at the end of the session. The phases of the Power Therapy from 8 to 14 will be repeated in cycles 2 times.

The access to the **Valgus Knee Therapy** mode requires only user's card to come closer the player. User can choose the program through the pc touchscreen, and, by getting his card closer to RFID player, the program will start by default.

Also in the Valgus Knee Therapy mode, user will be asked to perform any movements together with the liftings on the first device 1.

The Valgus Knee Therapy requires a spacer which is provided with the automated system 1.

The spacer has to be placed between the patient's knees, up to the femoral condyleses. Next, the operator will place the lateral motorized pistons (plungers) up to the ankles, laterally to the malleolus area. Then, he will close the pistons through the specific buttons, up to the lower limbs' alignment and/or stop if the patient gets pain.

At the beginning of the phase 8, a video will be shown on the monitor through the webcam located on the table: the video will be removed only at the end of the session. The phases of the Valgus Knee Therapy from 8 to 14 will be repeated in cycles 2 times.

The access to the **Varus Knee Therapy** mode requires only user's card to come closer the player. User can choose the program through the pc touchscreen, and, by getting his card closer to RFID player, the program will start by default.

Also in the Varus Knee Therapy mode, user will be asked to perform any movements together with the liftings on the first device 1.

The Varus Knee Therapy requires a spacer which is provided with the automated system 1.

The spacer has to be placed between the patient's ankles, up to the medial malleolus. Next, the operator will place the lateral motorized pistons up to the knees, in the femoral condyleses area. Then, he will close the pistons, through the specific buttons, up to the lower limbs' alignment and/or stopping if the patient gets pain.

At the beginning of the phase 8, a video will be shown on the monitor through the webcam located on the table: the video will be removed only at the end of the session. The phases of the Varus Knee Therapy phases from 8 to 14 will be repeated in cycles 2 times.

The management method also includes a patients' management software, connected to the therapy's implementation software which has been installed on the first device 1.

The management software will be used by administrative staff in order to undertake as follows:
a) administration: patients list, medical records (reports, X-ray, CAT scan, MRI, photograph, etc.);
b) financial administration: list of session/access to the device's therapies and treatments;
c) report: registration of patients' parameters and graphs of therapeutic's reporting, concerning the entire prescribed process and based on new tests recording patients' advancements and new parameters associated;
d) medical Centre's biographical data;
e) device remote management (the software can report if the device is busy and it can display the status of the table); and
f) network device configuration.

Aside from an intranet management inside each specific Centre where device(s) therapies have been held, an additional remote management software is being planned, for packages of treatments and invoicing. This option will be implemented at the time when partnership with "Guess Centers" are defined. It stems from the need of a simplified management for both parties involved, both on therapeutic side and on financial one.

This facility is composed of a software architecture applied to an external server and to a connected network infrastructure, on which a primary and unique Content Management System (CSM) is intended to be built.

Each specific device will be recognized and managed from a remote command through the software and network systems and will communicate with the central server. This remote command system will allow to monitor data-processing and constant communication between the device and the main server, manage devices' security against disruption through appropriate security software, block the device remotely, encrypt the way in the instrument through an accurate method of cryptography.

The following areas will be provided:
- device management area: reports regarding each device's position, alert regarding the operating conditions (ON/OFF), the numbers of therapies/access for each of the devices, etc; and
- financial administration area: each Guess Center will be allowed to gain access to its area and buy all packages of treatment it requires (special therapeutic boxes based on the various reported pathologies, including from 16 to 160 access for therapy, are planned).

This software infrastructure will allow a simple and efficient management, notably on a large-scale business. For example, we are referring to US entrepreneurial way, if an individual entrepreneur was willing to invest on several centers (franchising) with the same mark. He may be equipped with a number of devices situated in all the centers, so each patient will be able to log in wherever he prefers, since management is uniform and every patient's parameters are recorded and managed by a central server.

The present invention's method may be well described by the execution of standard therapy.

The patient will perform his ceilings' test supported by an operator, then log in the first device 1 through his card to be read and recognized by the RFID player and select the therapeutic program prescribed by his physician (a medical checkup being always required) through the monitor touchscreen. The software starts by default with an initial pause, to allow the patient to lie down on the table.

The screen interface acts as a guide since it will describe all therapy protocols through a picture and a textual description, then the treatment will start by default.

As the therapy proceeds, the software will support (guide) the patient to perform particular movements and postures to try by explaining them through the monitor.

Each phase includes a description, picture, start/stop acoustic signals and a countdown timer to indicate the movement timing, the specific protocol timing and the pause to respect.

It is a very important facility since the patient will be given the time needed to perform the protocols accurately. The end of the therapy will be notified through a particular acoustic signal.

After this first step, wherein the patient mainly works on deep fascia and muscular chain of head, neck and trunk up to upper and lower limbs, he goes to the second device 2 to continue his progressive therapy, working on other muscle chains, mainly to stretch inextensible or retracted anterior muscle chains, improve head and trunk's range of motion in extension, re-align pelvis and whole body and improve overall postural balance, the performed therapy on this second tool going on and operating to complement the one started on the first device 1; it will specifically work on the anterior flexor muscle chain, globally on its fascias up to the ends of lower limbs.

At the end of this therapy on the second device 2, the patient goes to the third device 3 and will work on posterior muscle chains in an anti-gravity position, namely standing (in the first device the patient was in a lying position). The therapy on the third device 3 will operate in an interactive mode, due to the presence of the four cameras allowing the patient to check the correct alignment of the spine and the pelvis and the lower limbs' torsion, in addition to the lateral alignment from toes to head, while performing the usual protocol. The third device 3 will allow to execute protocols starting from a comfortable position and gradually getting more intense, through a remote control directly managed by the patient. He will lift the chute (inclined plane), starting from a neutral position at 0 degrees and proceeding till a maximum back flexure position of the ankle, which can get up to a 35-degree limit whether under optimal conditions.

The final device is fundamental to treat the flexibility of the spine, which has been already warmed up and "prepared" by the previous devices, even if the last step is to come.

The patient goes to the fourth device 4: this device allows him to reach his maximum level in extension led only by his pain/trouble threshold and through an actuator operated by a remote control lifting up the patient who is lying on his back. The patient can decide the duration of the treatment: at first, the sessions will be very short, but they will gradually get longer, as soon as his endurance improves.

The last tool is the fifth device 5, improving the alignment of his lower limbs, then being mainly used for the treatment of knee varus and knee valgus, while he is balanced on the double chute (inclined plane), placed in a concave position in case of varus or a convex position in case of valgus.

Obviously, it will be the patient to decide the slope angles, according to his thresholds. The fifth device 5 will succeed in freeing (treating) the ankle which is in distress for a malalignment of lower limbs, thereby improving the alignment of hind foot (which is impossible to achieve through standard tools).

It is clear that the method has been projected to perform all the therapies gradually, through a conventional path for all the instruments, each of them contributing to the overall achievement independently.

Some pathologies that can be treated by the system are summarised as follows: varus/ valgus, scoliotic attitude, scoliosis hyperkyfosi, low back pain, neck pain, adhesive capsulitis (frozen shoulder), discopathy, disk herniation, coxalgy/ coxarthrosi, knee pain/ gonarthrosi, sports performance improvement, pubic pane, cns's degenerativ e diseases (parkinson's disease, multiple sclerosis, etc.), fybromialgia, musculoskel etal system's lower diseases in early stage.

To sum up, the first device 1 is composed of four distinct components:
1) **Therapeutic table:** muscle relaxation hardware, itself composed of:
   ∘ **Seat:** mechanical component that can be lifted while the patient is lying on the table;
   ∘ **Backrest:** mechanical part that can be lifted while the patient is lying on the table;
   ∘ **All in one Pc:** the hardware is equipped with a computer allowing the use of the management software. The monitor displays the ongoing method, allowing the patient to perform the protocols correctly;
   ∘ **Visor goggles:** enable the patient to isolate from his surroundings and checking his performance while totally relaxing;
   ∘ **RFID player:** identifies both the administrator and the patients and enables the access to the system through RFID card; and
   ∘ **LM01:** management software that enables patients to perform the procedures, also enabling the controller the access for other two procedures.
2) **Administration PC:** a computer, equipped with a management software, to log, modify, check the patients and to control the tables'status connected to the system from remote;
3) **Database Server:** it hosts a memory bank of the patients' data, of the tables' data, along with some configuration settings; and
4) **FTP Server:** this computer hosts an FTP server in which videos from the tables are listed. It is possible to manage both videos and the categories to which they belong from the management system. The videos uploaded on the PC connected to the table are sync with those on the FTP server (It is possible to sync both on demand and by default when the first device 1 starts to operate). By doing so, the same videos can be made available for each of the tables.

All the first device 1's components are interconnected through a LAN (local area network), which provides for reading, logging or modifying the data in the Server database, as well as to manage and sync the videos uploaded on the FTP server.

The table PC hosts the associated software, while the administrator PC one hosts the one related to users management.

Both the database and the FTP servers can be hosted separately on any PC connected to the grid, to free memory as much as possible.

The IT platform can operate in two possible ways: Autonomous or Networking.

The Autonomous mode provides for a table only, on which LM01 software and the server database are installed. It does not require an FTP server. The software connects and applies local database. It does not require the FTP server since videos are uploaded on the table's PC through a USB drive.

In Networking mode there is an administration PC and 1 or more therapeutic tables. The server database can be hosted independently, on one of the tables or on a server space created for this purpose. Both the tables and the administration PC are connected and use a remote database through a LAN and/or an Internet connection. The FTP server may also be hosted on the administration PC; by doing so, videos may be uploaded to this PC only, through the user management software, then each table just copies out them through LAN.

At any time it is possible to switch from one solution to another simply changing software configuration settings.

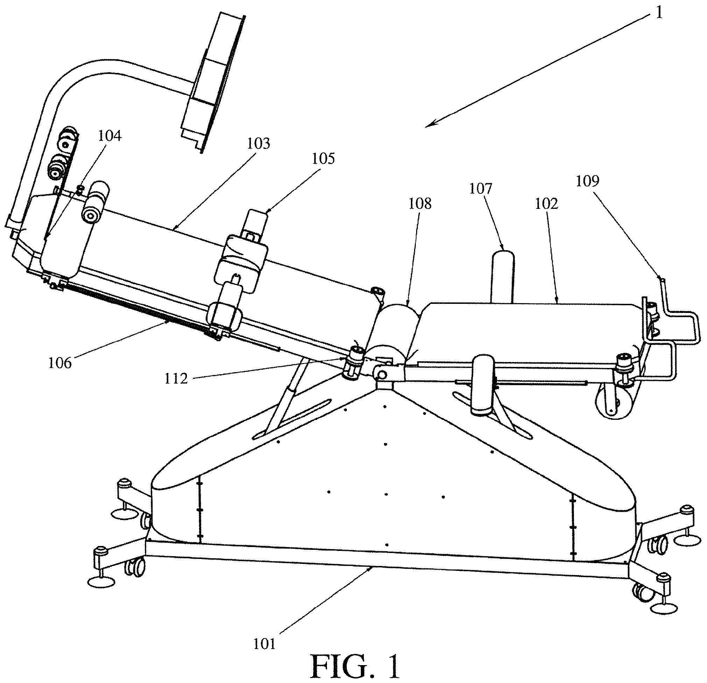

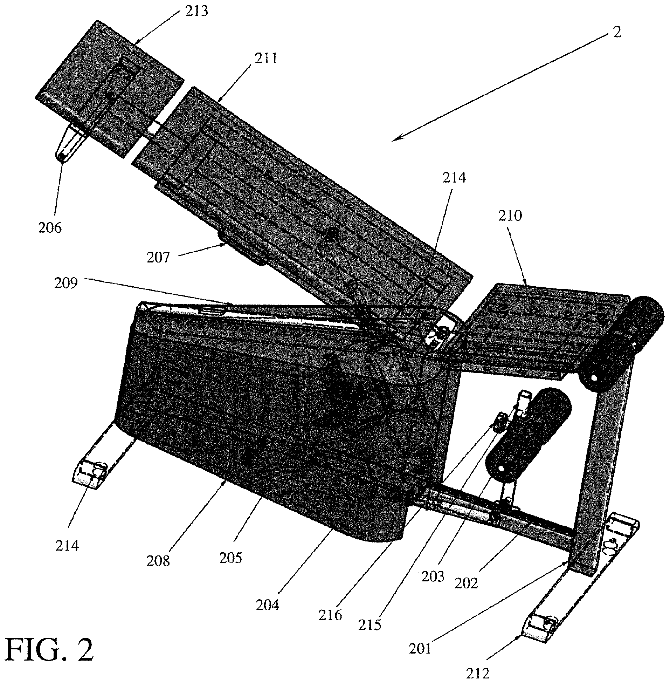

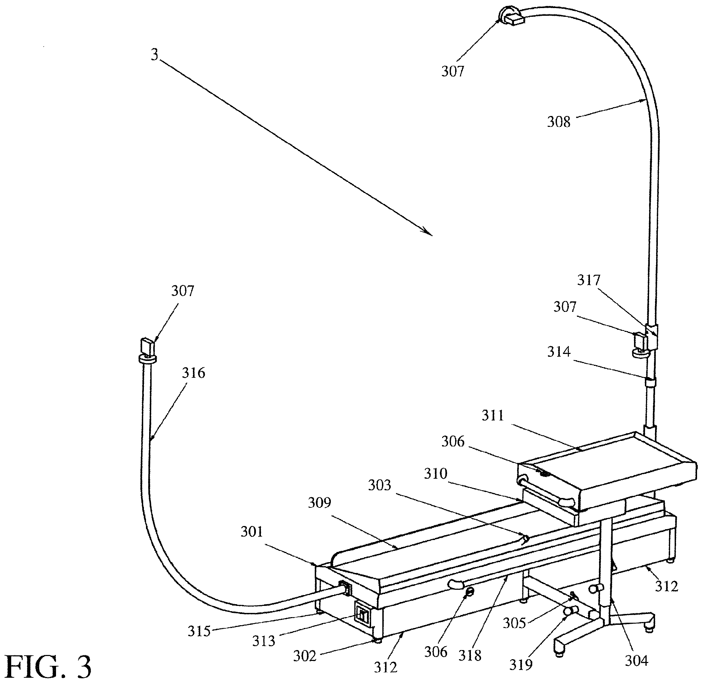

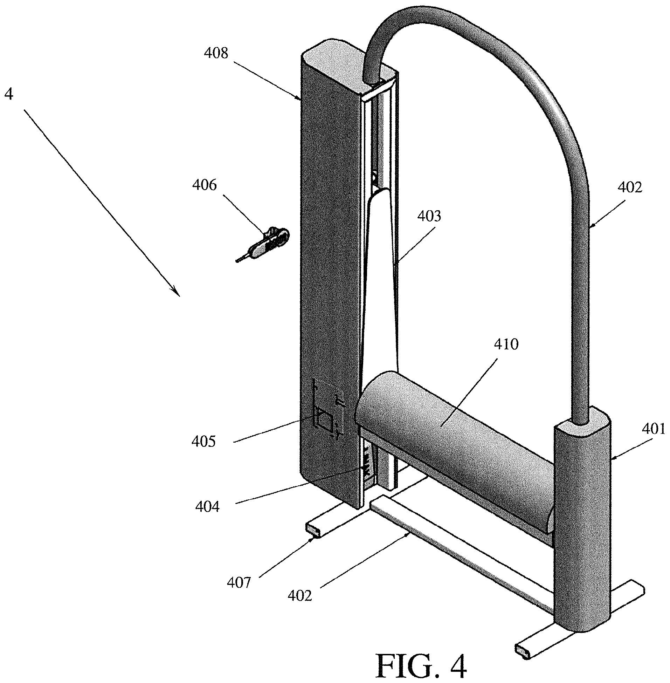

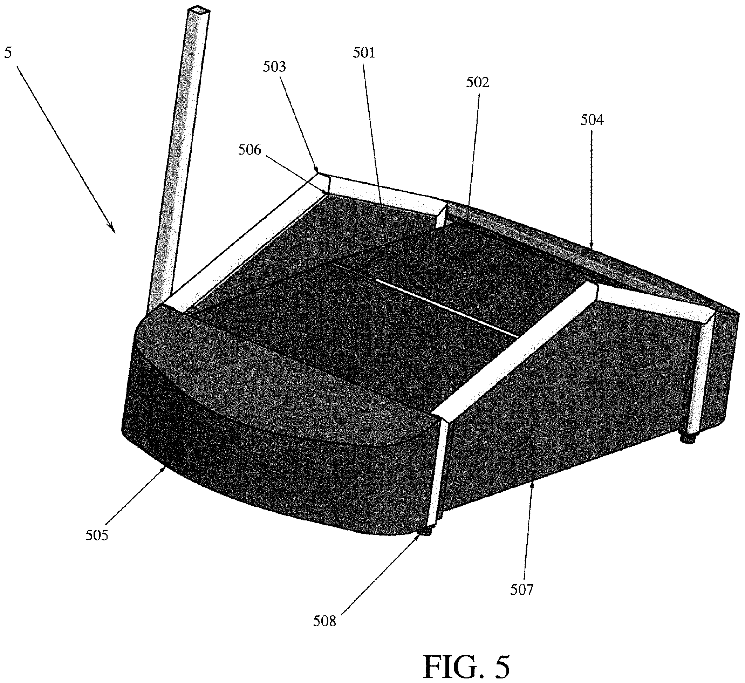

## Claims

1. Automated electro-medical system for treating, curing, analysing and monitoring a plurality of medical conditions related to spine and muscle-skeletal system, said electro-medical system comprising:
- a first device (1) of an automated and interactive posture bed type, the first device (1) comprising a personal computer, equipped with managing software and webcam, the personal computer configured for managing via software the bed movements and for managing the exercise path specific for every patient;
- a second device (2) of an automated and interactive posture bench;
- a third device (3) for an automated and interactive alignment of a spine;
- a fourth device (4) to automatically and interactively hydrating inter-vertebral disks; and
- a fifth device (5) for an automated and interactive alignment of lower limbs;
wherein the personal computer is configured for automatically guiding through the following exercise steps:
- the managing software guides the patient to the first device (1) and chooses a required exercise program, a managing program of the first device (1) is automatically started and allows the patient to be placed on the first device (1);
- after this exercise step, for deep fascia and muscular chain from head, neck and trunk to upper and lower limbs, the patient is guided to the second device (2), in order to work on other muscle chains, namely stretching anterior muscle chains, improving the range of motion of head and trunk in extension, aligning pelvis and whole body, and improving postural balance, the performed exercises on this second tool specifically working on the anterior flexor muscle chain, on its fascias globally till the ends of lower limbs;
- at the end of these exercises on the second device (2), the managing software guides the patient to the third device (3), said third device (3) allowing to work on posterior muscle chains in a standing position, the exercises performed on the third device (3) being operated in an interactive mode due to the presence of the four cameras which allow the patient to check the correct alignment of the spine and the pelvis, and the lower limb torsion, in addition to the lateral alignment from feet toe to head, said third device (3) is intended to lift the inclined plane from a neutral position at 0 degrees and progresses till a maximum back flexure position of the ankle, which can arrive at the 35-degree limit under optimum conditions;
- the managing software guides the patient to the fourth device (4), which allows the patient, to reach the maximum extension level of his spine, improving his elasticity, by lifting the patient, who rests on his back, from the ground;
- the managing software guides the patient to the fifth device (5), improving the alignment of his lower limbs, and then being mainly used for knee varus and valgus, while he is balanced on the double inclined plane, which is placed in a concave position in case of varus or in a convex position in case of valgus, the fifth device (5) further freeing the ankle, thereby improving the alignment of the rear foot part;
**characterized in that** through a screen interface, the managing program guides the patient, through a figure and text description of exercise protocols, and starts the exercises automatically, and guides the patient in performing specific movements and positions to be assumed, every phase containing description, image, acoustic signals for phase start/stop, and timer which, under countdown, signals the time for a movement, for the specific protocol to be performed and the related pause to be observed, the exercise end being signalled through a specific acoustic signal;

2. Automated electro-medical system according to claim 1, **characterized in that** said first device (1) comprises:
- a base (101), which is in particular a box with a metallic structure which contains therein an actuator to move a backrest (102), an actuator to move an abutment for lower limbs (103), an electronic part, namely a card and components;
- the backrest (102) rotating connected to the base (101);
- the abutment for lower limbs (103) rotating connected to the base (101) and to the backrest (102) ;
- a carriage for feet (104) operatively connected to the abutment for lower limbs (103);
- patient side pressing motors (105) to block a patient at his ankles or knees, sliding connected to the abutment for lower limbs (103) along a related sliding carriage (106); and
- two side rests for arms (107) connected to the backrest (102).

3. Automated electro-medical system according to claim 2, **characterized in that** said first device (1) further comprises:
- a central pillow (108) placed next to the connection fulcrum between base (101), backrest (102) and abutment for lower limbs (103); and
- two upper extendible handles (109) connected to the backrest (102) on the opposite side with respect to the central pillow (108),
- at least six adjustable resisting elastic elements (112) respectively connected as follows: two resistances connected between central pillow (108) and abutment for lower limbs (103); two resistances connected to the upper side of the backrest (102); two resistances connected to the carriage for feet (104).

4. Automated electro-medical system according to claim 1, **characterized in that** said second device (2) comprises:
- a supporting frame (201) equipped with a sliding guide (202) for bearing pillows (203) made of rubber;
- a base, which in particular is a box made of a metallic structure composed of a first half-carter (208) and a second half-carter (209), and which contains therein at least one actuator (205) to move a backrest (211), at least one actuator (204) to move the bearing pillows (203) made of rubber, in the carter (208, 209) a control unit being contained, to which a remote control is fastened for controlling the two actuators (204, 205), the backrest (211) being made in order to be made due to the motor inserted in the base (208, 209); and
- a sitting (210) which mirrors the backrest (211), with respect to a juncture and is fixed;
- the actuator (204), fastened to the supporting frame (201), adapted to move the bearing pillows (203), making them alternatively slide.

5. Automated electro-medical system according to claim 4, **characterized in that** said second device (2) further comprises:
- a headrest (213) connected to the backrest (211) ;
- a handle (207) connected to the backrest (211) ;
- two adjustable feet (214) equipped with respective plugs (212); and
- a mobile ankle-carrier (215) equipped with a respective adjusting knob (216).

6. Automated electro-medical system according to claim 1, **characterized in that** said third device (3) comprises:
- a motored inclined plane (301), possibly equipped with a sliding-preventing rubber plane (309) and a supporting frame (302);
- a containing structure composed of a carter (312), a curved carter (303), with related small adjustable feet (315);
- an actuator contained inside the containing structure;
- a monitor (311);
- a visor (310); and
- four cameras (307) which allows the patient to wholly autonomously obtain the alignment of the muscle-skeletal system, the first camera shooting the spine from above, the second camera shooting the side vision of the torso, the third camera sending back the side vision of the whole body and the fourth camera shooting the exact alignment of lower limbs, wherein the visor (310) is designed to project an image of the patient's body shot from different perspectives through the four cameras (307) .

7. Automated electro-medical system according to claim 1, **characterized in that** said fourth device (4) comprises:
- two pillars (401,408) connected to a plane with curved pillow (410);
- an actuator (404) inserted in the carter (408) of the right pillar, said actuator (404), actuated by a remote control, (406), allowing to gradually lift the plane (410) which connects the two pillars (401,408), a patient, by resting on the plane (410), lifting and lowering the plane (410) till a greater elasticity of the spine is progressively obtained, with benefits for inter-vertebral disks.

8. Automated electro-medical system according to claim 1, **characterized in that** said fifth device (5) comprises:
- a supporting structure (503) with possible adjustable small feet (508);
- two inclined planes (502), on which the patient is placed standing when the base (501) is horizontal, the two separate inclined planes (502), one for each foot, being controlled by a pushbutton panel which actuates an actuator placed below the two inclined planes (502), the actuator taking up and/or down the two inclined planes (502), forming an acute angle by rising upwards or descending downwards, thereby strongly stressing the patient's ankle.

## Patentansprüche

1. Automatisiertes elektromedizinisches System für die Behandlung, die Heilung, die Untersuchung und die Überwachung einer Vielzahl medizinischer Erkrankungen der Wirbelsäule und des Bewegungsapparates, das genannte elektromedizinische System enthält:
- eine erste Vorrichtung (1), eine automatisierte und interaktive Haltungsliege, die erste Vorrichtung (1) enthält einen Personal Computer, der mit Verwaltungssoftware und Webcam ausgestattet ist, der Personal Computer wurde konfiguriert, um die Bewegungen der Liege über Software zu steuern und die spezifische Therapie für jeden Patienten zu verwalten;
- eine zweite Vorrichtung (2), eine automatisierte und interaktive Haltungsbank;
- eine dritte Vorrichtung (3) für eine automatisierte und interaktive Ausrichtung der Wirbelsäule;
- eine vierte Vorrichtung (4) zur automatischen und interaktiven Hydratation der Bandscheiben; und
- eine fünfte Vorrichtung (5) für eine automatisierte und interaktive Ausrichtung der Beine;
wobei der Personal Computer konfiguriert wurde, um eine automatische Führung durch die folgenden Therapiephasen auszuführen:
- Die Verwaltungssoftware führt den Patienten zur ersten Vorrichtung (1) und wählt ein verordnetes Therapieprogramm, ein Verwaltungsprogramm der ersten Vorrichtung (1) startet automatisch und ermöglicht es dem Patienten, sich auf der ersten Vorrichtung (1) zu positionieren;
- Nach dieser Therapiephase für die hinteren Bänder und Faszien des Rumpfes, Hals, Kopf, Beine und Arme wird der Patient zu einer zweiten Vorrichtung (2) geführt, um an den anderen Muskelketten zu arbeiten, d. h. die vorderen hypoextensiblen oder retraktierten Muskeln dehnbar zu machen, die Bewegungsweite der Ausdehnung des Rumpfes und des Kopfes zu verbessern, das Becken und den gesamten Körper auszurichten und das Haltungsgleichgewicht zu verbessern, die auf diesem zweiten Instrument ausgeführte Therapie arbeitet spezifisch an der vorderen Muskelkette der Beugung, global an ihren Faszien bis zum Ende der Beine;
- Am Ende dieser Therapie auf der zweiten Vorrichtung (2) führt die Verwaltungssoftware den Patienten zur dritten Vorrichtung (3), die genannte dritte Vorrichtung (3) ermöglicht es, an den hinteren Ketten in stehender Position zu arbeiten, die auf der dritten Vorrichtung (3) ausgeführte Therapie wird durch die Anwesenheit der vier Kameras interaktiv ausgeführt, die es dem Patienten ermöglichen, die korrekte Ausrichtung der Wirbelsäule und des Beckens sowie die Torsion der Beine und die Ausrichtung von der Fußspitze bis zum Kopf von der Seite zu kontrollieren, die genannte dritte Vorrichtung (3) ist vorgesehen, um die schräge Ebene von einer neutralen Position von 0 Grad anzuheben und bis zur maximalen dorsalen Beugung des Knöchels fortzufahren, die in optimalen Bedingungen die Grenze von 35 Grad erreichen kann;
- Die Verwaltungssoftware führt den Patienten zur vierten Vorrichtung (4), die es dem Patienten ermöglicht, den maximalen Ausdehnungsgrad der Wirbelsäule zu erreichen und die Dehnbarkeit durch Anheben des Patienten vom Boden, der auf dem Rücken liegt, zu verbessern;
- Die Verwaltungssoftware führt den Patienten zur fünften Vorrichtung (5), wo sie die Optimierung der Ausrichtung der Beine ausführt und daher vorwiegend für Achsenfehlstellungen der Knie (Genu varum, Genu valgum) verwendet wird, während der Patient auf der doppelten schrägen Ebene im Gleichgewicht ist, die im Falle des Genu varum in konkaver Position und im Falle des Genu valgum in konvexer Position positioniert wird, die fünfte Vorrichtung (5) befreit außerdem den Knöchel und verbessert auf diese Weise die Ausrichtung der Fußrückseite;
und ist **dadurch gekennzeichnet, dass** das Verwaltungsprogramm den Patienten mithilfe der Schnittstelle auf dem Bildschirm durch eine Abbildung und eine Textbeschreibung der Therapieprotokolle führt und die Therapie automatisch startet und den Patienten bei der Ausführung der spezifischen Bewegungen und der einzunehmenden Positionen führt, jede Phase enthält die Beschreibung, das Bild, die akustischen Signale für Start/Ende der Phase und den Timer, der im Countdown die Zeit der Bewegung, des spezifischen auszuführenden Protokolls und der entsprechenden zu beachtenden Pause anzeigt, das Ende der Therapie wird durch ein spezifisches akustisches Signal angezeigt.

2. Automatisiertes elektromedizinisches System gemäß Patentanspruch 1, das **dadurch gekennzeichnet ist, dass** die genannte erste Vorrichtung (1) Folgendes enthält:
- eine Basis (101), die insbesondere ein Gehäuse mit einer Metallstruktur ist, das in seinem Inneren einen Antrieb enthält, um eine Rückenlehne (102) zu bewegen, einen Antrieb, um eine Beinstütze (103) zu bewegen, einen elektronischen Teil, d. h. eine Steuerkarte und Komponenten;
- die Rückenlehne (102) ist drehend mit der Basis (101) verbunden;
- die Beinstütze (103) ist drehend mit der Basis (101) und der Rückenlehne (102) verbunden;
- ein Fußwagen (104) ist für den Betrieb mit der Beinstütze (103) verbunden;
- Motoren für seitliche Niederhalter (105), um einen Patienten in Höhe der Knöchel oder Knie zu blockieren, die mit der Beinstütze (103) gleitend längs eines entsprechenden Gleitwagens (106) verbunden sind; und
- zwei seitliche Armstützen (107), die mit der Rückenlehne (102) verbunden sind.

3. Automatisiertes elektromedizinisches System gemäß Patentanspruch 2, das **dadurch gekennzeichnet ist, dass** die genannte erste Vorrichtung (1) außerdem Folgendes enthält:
- ein mittleres Kissen (108), das am Schwerpunkt der Verbindung zwischen Basis (101), Rückenlehne (102) und Beinstütze (103) angebracht wurde
- zwei obere ausziehbare Griffe (109), die mit der Rückenlehne (102) von der entgegengesetzten Seite gegenüber dem mittleren Kissen (108) verbunden sind;
- mindestens sechs verstellbare Widerstandsschläuche (112), die jeweils wie folgt verbunden sind: Zwei Widerstandsschläuche sind zwischen dem mittleren Kissen (108) und der Beinstütze (103) verbunden; zwei Widerstandsschläuche sind auf der Oberseite der Rückenlehne (102) verbunden; zwei Widerstandsschläuche sind mit dem Fußwagen (104) verbunden.

4. Automatisiertes elektromedizinisches System gemäß Patentanspruch 1, das **dadurch gekennzeichnet ist, dass** die genannte zweite Vorrichtung (2) Folgendes enthält:
- ein Gestell (201), das mit einer Gleitführung (202) für die Stützkissen (203) aus Gummi ausgestattet ist;
- eine Basis, die insbesondere ein Gehäuse ist, das mit einer Metallstruktur ausgeführt wurde, welches aus einer ersten Gehäusehälfte (208) und einer zweiten Gehäusehälfte (209) besteht und welches in seinem Inneren mindestens einen Antrieb (205) für die Bewegung einer Rückenlehne (211) und mindestens einen Antrieb (204) für die Bewegung der Stützkissen (203) aus Gummi enthält, im Gehäuse (208, 209) ist eine Bedientafel enthalten, an der eine Fernbedienung für die Steuerung der beiden Antriebe (211) befestigt ist, die so gefertigt wurde, dass sie dank des Motors bewegt werden kann, der in die Basis (208, 209) eingesetzt wurde; und
- eine spiegelbildliche Sitzfläche (210) zur Rückenlehne (211) gegenüber dem Gelenk, das fest ist;
- Der Antrieb (204), der am Gestell (201) befestigt ist, dient dazu, die Stützkissen (203) zu bewegen, indem er sie vor- und rückwärts gleiten lässt.

5. Automatisiertes elektromedizinisches System gemäß Patentanspruch 4, das **dadurch gekennzeichnet ist, dass** die genannte zweite Vorrichtung (2) außerdem Folgendes enthält:
- eine Kopfstütze (213), die mit der Rückenlehne (211) verbunden ist;
- einen Griff (207), der mit der Rückenlehne (211) verbunden ist;
- zwei verstellbare Stützfüße (214), die mit entsprechenden Verschlüssen (212) ausgestattet sind; und
- eine bewegliche Knöchelstütze (215), die mit einem entsprechenden Stellgriff (216) ausgestattet ist.

6. Automatisiertes elektromedizinisches System gemäß Patentanspruch 1, das **dadurch gekennzeichnet ist, dass** die genannte dritte Vorrichtung (3) Folgendes enthält:
- eine angetriebene schräge Plattform (301), die ggf. mit einer rutschfesten Gummiebene (309) und einem Gestell (302) ausgestattet ist;
- eine Rückhaltestruktur, die aus einem Gehäuse (312), einem gebogenen Gehäuse (303) mit entsprechenden verstellbaren Stützfüßen (315) besteht;
- einen Antrieb, der im Inneren der Rückhaltestruktur enthalten ist;
- einen Monitor (311);
- ein Sichtgerät (310); und
- vier Kameras (307), die es dem Patienten ermöglichen, die Ausrichtung des Bewegungsapparates vollkommen autonom zu erhalten, die erste Kamera nimmt die Wirbelsäule von oben auf, die zweite Kamera nimmt die Seitenansicht des Oberkörpers auf, die dritte Kamera sendet die Seitenansicht des gesamten Körpers zurück, und die vierte Kamera nimmt die genaue Ausrichtung der Beine auf, wobei das Sichtgerät (310) dazu entwickelt wurde, ein Bild des Körpers des Patienten zu projizieren, das aus verschiedenen Perspektiven durch die vier Kameras (307) aufgenommen wurde.

7. Automatisiertes elektromedizinisches System gemäß Patentanspruch 1, das **dadurch gekennzeichnet ist, dass** die genannte vierte Vorrichtung (4) Folgendes enthält:
- vier Säulen (401,408), die durch eine Ebene mit gewölbtem Kissen (410) verbunden sind;
- einen Antrieb (404), der im Gehäuse (408) der rechten Säule eingesetzt wurde, der genannte Antrieb (404), der durch eine Fernbedienung (406) aktiviert wird, ermöglicht es, die Ebene (410), die die beiden Säulen (401,408) verbindet, schrittweise mit einem auf der Ebene (410) liegenden Patienten anzuheben, die Ebene (410) anzuheben und abzusenken, bis schrittweise eine höhere Dehnbarkeit der Wirbelsäule mit Vorteilen für die Bandscheiben erhalten wird.

8. Automatisiertes elektromedizinisches System gemäß Patentanspruch 1, das **dadurch gekennzeichnet ist, dass** die genannte fünfte Vorrichtung (5) Folgendes enthält:
- eine Trägerstruktur (503), ggf. mit verstellbaren Stützfüßen (508);
- zwei schräge Ebenen (502), auf denen sich der Patient stehend positioniert, wenn die Basis (501) horizontal ist, die zwei getrennten schrägen Ebenen (502), eine pro Fuß, werden durch eine Bedientafel gesteuert, die einen Antrieb aktiviert, der unter den beiden Ebenen (502) angebracht wurde, der Antrieb hebt die beiden Ebenen (502) an und senkt sie, wobei sie durch die Auf- und Abwärtsbewegung einen spitzen Winkel bilden und auf diese Weise stark den Knöchel des Patienten belasten.

## Revendications

1. Système électromédical pour le traitement, le soin, l'analyse et le suivi d'une pluralité de pathologies médicales relatives à la colonne vertébrale et à l'appareil locomoteur; ce système électromédical comprend:
- un premier dispositif (1), à savoir un lit postural automatisé et interactif, constitué d'un ordinateur doté d'un logiciel de gestion et d'une webcam; l'ordinateur est configuré pour gérer via le logiciel les mouvements du lit et le parcours thérapeutique spécifique de chaque patient;
- un deuxième dispositif (2), à savoir une banquette posturale automatisée et interactive;
- un troisième dispositif (3) pour un alignement automatisé et interactif de la colonne vertébrale;
- un quatrième dispositif (4) pour hydrater automatiquement et de manière interactive les disques intervertébraux; et
- un cinquième dispositif (5) pour un alignement automatisé et interactif des membres inférieurs;
où l'ordinateur est configuré pour exécuter un guidage automatique à travers les phases thérapeutiques suivantes:
- le logiciel de gestion guide le patient vers le premier dispositif (1) et choisit un programme thérapeutique prescrit; un programme de gestion du premier dispositif (1) est alors automatiquement lancé; il permet au patient de se positionner sur le premier dispositif (1);
- après cette phase de thérapie des chaînes et des fascias arrière du tronc, cou, tête, membres inférieurs et supérieurs, le patient est guidé vers le deuxième dispositif (2), pour travailler sur d'autres chaînes musculaires, à savoir étirer les chaînes musculaires avant hypo-extensibles ou contracturées, améliorer l'amplitude de mouvement de l'extension du tronc et de la tête, aligner le bassin et tout le corps ainsi qu'améliorer le rééquilibrage postural; la thérapie réalisée sur ce deuxième dispositif permet de travailler de manière spécifique sur la chaîne musculaire avant de flexion et, de manière globale, sur ses fascias jusqu'à l'extrémité des membres inférieurs;
- à la fin de cette thérapie sur le deuxième dispositif (2), le logiciel de gestion guide le patient vers le troisième dispositif (3) qui permet de travailler sur les chaînes arrière en restant debout; la thérapie réalisée sur ce troisième dispositif (3) est effectuée de manière interactive grâce à la présence de quatre caméras qui permettent au patient de contrôler l'alignement correct de la colonne vertébrale et du bassin, la torsion des membres inférieurs ainsi que l'alignement latéral de la pointe des pieds par rapport à la tête; ce troisième dispositif (3) est prévu pour soulever le plan incliné à partir d'une position neutre de 0 degré en progressant jusqu'à la position maximale de flexion dorsale de la cheville qui peut atteindre la limite de 35 degrés en conditions optimales;
- le logiciel de gestion guide le patient vers le quatrième dispositif (4) qui permet d'atteindre le niveau maximal d'extension de la colonne vertébrale, en améliorant son élasticité, au moyen du soulèvement du patient qui est couché sur le dos;
- le logiciel de gestion guide le patient vers le cinquième dispositif (5) qui aide à perfectionner l'alignement des membres inférieurs; ce dispositif est utilisé principalement pour les genoux en varus ou en valgus, le patient reste en équilibre sur le double plan incliné qui est placé en position concave en cas de genoux en varus ou bien en position convexe en cas de genoux en valgus; par ailleurs, le cinquième dispositif (5) libère la cheville en améliorant ainsi l'alignement de la partie arrière du pied;
**caractérisé en ce que**, à travers l'interface sur l'écran, le programme de gestion guide le patient, à l'aide d'une figure et d'une description textuelle des protocoles thérapeutiques; ensuite, il lance automatiquement la thérapie et il guide le patient dans l'exécution des mouvements spécifiques et des positions à prendre; chaque phase est accompagnée d'une description, d'une image, de signaux sonores de début/fin de la phase et d'un temporisateur qui, avec un compte à rebours, signale le temps du mouvement du protocole spécifique à suivre et la pause relative à respecter; la fin de la thérapie est signalée par un signal sonore spécifique.

2. Système électromédical automatisé, selon la revendication 1, **caractérisé en ce que** le premier dispositif (1) comprend:
- une base (101), en particuliers une boîte avec une structure métallique, renfermant un actionneur pour déplacer le dossier (102), un actionneur pour déplacer le support pour les membres inférieurs (103), une partie électronique, à savoir une carte et ses composants;
- le dossier (102) relié de manière rotative à la base (101);
- le support pour les membres inférieurs (103) relié de manière rotative à la base (101) et au dossier (102);
- un chariot pour les pieds (104) relié opérationnellement au support pour les membres inférieurs (103);
- des moteurs presseurs latéraux (105) pour bloquer un patient à la hauteur des chevilles ou des genoux, reliés au support pour les membres inférieurs (103) de manière coulissante le long d'un chariot coulissant relatif (106); et
- deux supports latéraux pour les bras (107) reliés au dossier (102).

3. Système électromédical automatisé, selon la revendication 2, **caractérisé en ce que** le premier dispositif (1) comprend aussi:
- un coussin central (108) situé au niveau du centre de connexion entre la base (101), le dossier (102) et le support pour les membres inférieurs (103)
- deux poignées extensibles supérieures (109) reliées au dossier (102) du côté opposé par rapport au coussin central (108);
- au moins six tubes élastiques de résistance réglables (112) reliés respectivement comme suit: deux tubes de résistance reliés entre le coussin central (108) et le support pour les membres inférieurs (103); deux tubes de résistance reliés au côté supérieur du dossier (102); deux tubes de résistance reliés au chariot pour les pieds (104).

4. Système électromédical automatisé, selon la revendication 1, **caractérisé en ce que** le deuxième dispositif (1) comprend:
- un châssis de support (201) doté d'une glissière (202) pour les coussins de soutien (203) en caoutchouc;
- une base, en particuliers une boîte réalisée avec une structure métallique, composée d'un premier demi-protecteur (208) et d'un second demi-protecteur (209), renfermant au moins un actionneur (205) pour déplacer le dossier (211), au moins un actionneur (204) pour déplacer les coussins de soutien (203) en caoutchouc; le protecteur (208, 209) contient une unité de commande à laquelle est reliée une télécommande permettant de contrôler les deux actionneurs (204, 205); le dossier (211) est réalisé de sorte à pouvoir être déplacé par le moteur inséré dans la base (208, 209); et
- un siège (210) spéculaire au dossier (211), par rapport à la jonction qui est fixe;
- l'actionneur (204), ancré au châssis de support (201), est apte à déplacer les coussins de soutien (203), en les faisant glisser en avant et en arrière.

5. Système électromédical automatisé, selon la revendication 4, **caractérisé en ce que** le deuxième dispositif (2) comprend aussi:
- un appui-tête (213) relié au dossier (211);
- une poignée (207) reliée au dossier (211);
- deux pieds réglables (214) dotés de bouchons relatifs (212); et
- un repose-cheville mobile (215) doté d'un bouton de réglage (216).

6. Système électromédical automatisé, selon la revendication 1, **caractérisé en ce que** le troisième dispositif (1) comprend:
- une plateforme inclinée motorisée (301), éventuellement dotée d'un plan caoutchouté antiglisse (309) et d'un châssis de support (302);
- une structure de retenue composée d'un protecteur (312), d'un protecteur courbe (303), avec les pieds réglables relatifs (315);
- un actionneur renfermé dans la structure de retenue;
- un écran (311);
- un casque de vision (310); et
- quatre caméras (307) permettant au patient d'obtenir l'alignement de l'appareil locomoteur de manière tout à fait autonome; la première caméra encadre du haut la colonne vertébrale, la deuxième caméra encadre la vision latérale du torse, la troisième caméra donne la vision latérale de tout le corps et la quatrième caméra encadre l'alignement correct des membres inférieurs; où le casque de vision (310) a été conçu pour projeter une image du corps du patient prise à partir de différentes perspectives à l'aide des quatre caméras (307).

7. Système électromédical automatisé, selon la revendication 1, **caractérisé en ce que** le quatrième dispositif (1) comprend aussi:
- deux piliers (401,408) reliés par un plan au coussin courbe (410);
- un actionneur (404) inséré dans le protecteur (408) du pilier de droite; cet actionneur (404) est commandé par une télécommande (406) qui permet de soulever graduellement le plan qui relie les deux piliers (401,408); le patient est allongé sur le plan (410) qui est soulevé et baissé progressivement jusqu'à obtenir une plus grande élasticité de la colonne vertébrale et donc des bénéfices pour les disques intervertébraux.

8. Système électromédical automatisé, selon la revendication 1, **caractérisé en ce que** le cinquième dispositif (1) comprend:
- une structure de support (503) avec éventuellement des pieds réglables (508);
- deux plans inclinés (502), sur lesquels le patient se positionne debout quand la base (501) est en position horizontale; les deux plans inclinés (502) sont séparés, un pour chaque pied; ils sont commandés par une boîte à boutons qui active l'actionneur situé sous les deux plans (502); l'actionneur positionne en haut et/ou en bas les deux plans (502) jusqu'à former un angle aigu en montant vers le haut ou en descendant vers le bas, en sollicitant ainsi fortement la cheville du patient.
